# EUROPEAN PATENT APPLICATION

(11) **EP 4 549 939 A1**
(43) Date of publication of application: **07.05.2025**
(21) Application number: 23831495.9
(22) Date of filing: 28.06.2023
(51) Int. Cl.: G01N 33/543, G01N 33/569

(54) **TEST METHOD, TEST REAGENT, AND TEST KIT**

(30) Priority: 30.06.2022 JP 2022105494
(71) Applicant: Sekisui Medical Co., Ltd., Tokyo 103-0027 (JP)
(72) Inventor: KOHNO, Keigo, Tokyo 103-0027 (JP); YAJI, Shohei, Tokyo 103-0027 (JP); ITO, Shizuka, Tokyo 103-0027 (JP); OKUYAMA, Shinya, Tokyo 103-0027 (JP); KAWASAKI, Joji, Tokyo 103-0027 (JP)
(74) Representative: Ter Meer Steinmeister & Partner
(86) International application number: PCT/JP2023/023931
(87) International publication number: WO 2024/005055

(57) **Abstract**

A qualitative or quantitative test method to detect a substance to be detected contained in a specimen, the test method including a preparation step of preparing a test sample from the specimen, and a test step of testing at least part of the test sample with a reagent to detect each of a first substance to be detected and a second substance to be detected, in which the first substance to be detected has an isoelectric point of 10.0 or more, and in any one or both of the preparation step and the test step, the first substance to be detected is brought into contact with any one or both of a glass-made member and a nitrocellulose-made member in the presence of a cationic substance. In this test method, two or more substances to be detected including a substance having an isoelectric point of 10.0 or more can be detected with high sensitivity using the same specimen while suppressing a decrease in measurement sensitivity.

## Description

### Technical Field

The present invention relates to a test method, a test reagent, and a test kit.

Priority is claimed on Japanese Patent Application No. 2022-105494, filed June 30, 2022, the content of which is incorporated herein by reference.

### Background Art

The SARS-CoV-2 is a virus that causes coronavirus disease 2019 (COVID-19). At present, COVID-19 is prevalent worldwide, and many people have been infected. Since the symptoms of COVID-19 are similar to those of respiratory infections caused by influenza, adenovirus, RS virus, and the like, it is difficult to discriminate the causative virus of infection from the symptoms.

For the diagnosis of a respiratory infection, a reagent represented by a rapid diagnostic reagent based on immunochromatography is widely used. In using such a diagnostic reagent, it is necessary to collect a specimen from the nasopharynx or the like of a patient with a cotton swab. In a situation where an infection is prevalent, to specify the causative virus of the infection, collecting a specimen from the nasal cavity or the pharynx for each pathogen of a test target increases the risk of droplet exposure to a medical worker by a cough or a sneeze of the patient.

Patent Document 1 describes a respiratory infection test method of preparing a test sample suitable for a plurality of respiratory infection tests by treating a specimen selected from a nasal aspirate, a nasal swab solution, and a pharyngeal swab solution with a specimen treatment solution, testing part of the test sample using a first test tool of an immunochromatography method for a first respiratory infection test, and testing part of the test sample using a second test tool of an immunochromatography method for a second respiratory infection test different from the first respiratory infection test, and a method of preparing a test sample suitable for a plurality of respiratory infection tests and performing a test for a plurality of types of viruses.

### Citation List

### Patent Document

[Patent Document 1]
Japanese Unexamined Patent Application, First Publication No. 2008-164403

### Summary of Invention

### Technical Problem

It has not been clarified whether the test method described in Patent Document 1 can be applied to testing for new viruses such as SARS-CoV-2.

The present inventors have found that a nucleocapsid protein, which is a detection target in the immunoassay of SARS-CoV-2, has an isoelectric point of 10 or more, and in a case where a substance having an isoelectric point of 10.0 or more is brought into contact with a filter for specimen filtration or a glass-made member in a reagent, the substance having an isoelectric point of 10.0 or more is adsorbed on the glass-made member, and the measurement sensitivity is decreased (Analytical Examples 1 to 3). So far, a method for detecting two or more substances to be detected including a substance having an isoelectric point of 10.0 or more with high sensitivity using the same specimen while suppressing such a decrease in measurement sensitivity has not been discussed.

An object of the present invention is to provide a test method capable of detecting two or more substances to be detected including a substance having an isoelectric point of 10.0 or more with high sensitivity using the same specimen while suppressing a decrease in measurement sensitivity.

### Solution to Problem

The present invention includes the following aspects.
[1] A qualitative or quantitative test method to detect a substance to be detected contained in a specimen, the test method including:
   a preparation step of preparing a test sample from the specimen, and
   a test step of testing at least part of the test sample with a reagent to detect each of a first substance to be detected and a second substance to be detected,
   in which the first substance to be detected has an isoelectric point of 10.0 or more, and
   in any one or both of the preparation step and the test step, the first substance to be detected is brought into contact with any one or both of a glass-made member and a nitrocellulose-made member in a presence of a cationic substance.
[2] The test method according to [1], in which the reagent includes a sample supply part and a labeled reagent holding part, and any one or both of the sample supply part and the labeled reagent holding part include the glass-made member.
[3] A qualitative or quantitative test method to detect a substance to be detected contained in a specimen, the test method including
   a preparation step of preparing a test sample from the specimen,
   a first test step of testing at least part of the test sample with a first reagent to detect each of a first substance to be detected and a second substance to be detected, and
   a second test step of testing part of the test sample with a second reagent to detect the second substance to be detected,
   in which the first substance to be detected has an isoelectric point of 10.0 or more, and
   in any one or both of the preparation step and the first test step, the first substance is brought into contact with any one or both of a glass-made member and a nitrocellulose-made member in a presence of a cationic substance.
[4] The test method according to [3], in which the first reagent includes a sample supply part and a labeled reagent holding part, and any one or both of the sample supply part and the labeled reagent holding part include the glass-made member.
[5] The test method according to any one of [1] to [4], in which the preparation step includes a step of suspending the specimen in a specimen-diluting liquid.
[6] The test method according to [5], in which the cationic substance is contained in the specimen-diluting liquid.
[7] The test method according to any one of [1] to [6], in which the preparation step includes a step of filtering the test sample with a filter.
[8] The test method according to any one of [1] to [7], in which the glass-made member is a filter for specimen filtration including glass fibers.
[9] The test method according to any one of [1] to [8], in which the cationic substance is at least one selected from the group consisting of a metal salt, a cationic polymer, a cationic peptide, and a cationic surfactant.
[10] The test method according to any one of [1] to [9], in which the substance having an isoelectric point of 10.0 or more is derived from a respiratory infection virus.
[11] The test method according to [10], in which the respiratory infection virus is a coronavirus.
[12] The test method according to [11], in which the coronavirus is SARS-CoV-2.
[13] A reagent including
   a first labeled reagent in which a first binding substance capable of binding to a first substance to be detected is labeled with a labeling substance,
   a second labeled reagent in which a second binding substance capable of binding to a second substance to be detected is labeled with a labeling substance,
   a first detection part in which a first solid phase reagent capable of binding to the first substance to be detected is immobilized,
   a second detection part in which a second solid phase reagent capable of binding to the second substance to be detected is immobilized, and
   any one or both of a glass-made member and a nitrocellulose-made member arranged upstream of the first detection part in a development direction of a development liquid containing a specimen,
   in which the first substance has an isoelectric point of 10.0 or more, and
   the development liquid brought into contact with any one or both of the glass-made member and the nitrocellulose-made member contains a cationic substance.
[14] The reagent according to [13], in which the reagent includes a sample supply part and a labeled reagent holding part, and any one or both of the sample supply part and the labeled reagent holding part include the glass-made member.
[15] The reagent according to [13] or [14], in which the glass-made member is a filter for specimen filtration including glass fibers.
[16] The reagent according to any one of [13] to [15], in which the substance having an isoelectric point of 10.0 or more is derived from a respiratory infection virus.
[17] The reagent according to [16], in which the respiratory infection virus is a coronavirus.
[18] The reagent according to [17], in which the coronavirus is SARS-CoV-2.
[19] The reagent according to any one of [13] to [18], in which the cationic substance is at least one selected from the group consisting of a metal salt, a cationic polymer, a cationic peptide, and a cationic surfactant.
[20] A qualitative or quantitative test method of a substance to be detected contained in a specimen using the reagent according to any one of [13] to [19].
[21] A test kit including
   a reagent,
   in which the reagent includes a first labeled reagent in which a first binding substance capable of binding to a first substance to be detected is labeled with a labeling substance, a second labeled reagent in which a second binding substance capable of binding to a second substance to be detected is labeled with a labeling substance, a first detection part in which a first solid phase reagent capable of binding to the first substance to be detected is immobilized, and a second detection part in which a second solid phase reagent capable of binding to the second substance to be detected is immobilized,
   the test kit includes any one or both of a glass-made member and a nitrocellulose-made member arranged upstream of the first detection part in a development direction of a development liquid containing a specimen,
   the first substance to be detected has an isoelectric point of 10.0 or more, and
   the development liquid brought into contact with any one or both of the glass-made member and the nitrocellulose-made member contains a cationic substance.
[22] A test kit including
   a specimen filtration filter, and
   a reagent,
   in which the reagent includes a first labeled reagent in which a first binding substance capable of binding to a first substance to be detected is labeled with a labeling substance, a second labeled reagent in which a second binding substance capable of binding to a second substance to be detected is labeled with a labeling substance, a first detection part in which a first solid phase reagent capable of binding to the first substance to be detected is immobilized, and a second detection part in which a second solid phase reagent capable of binding to the second substance to be detected is immobilized,
   the test kit includes any one or both of a glass-made member and a nitrocellulose-made member arranged upstream of the first detection part in a development direction of a development liquid containing any one or both of a specimen of the specimen filtration filter and a specimen of the reagent,
   the first substance to be detected has an isoelectric point of 10.0 or more, and
   the development liquid brought into contact with any one or both of the glass-made member and the nitrocellulose-made member contains a cationic substance.
[23] The test kit according to [21] or [22], in which the reagent includes a sample supply part and a labeled reagent holding part, and any one or both of the sample supply part and the labeled reagent holding part include the glass-made member.
[24] The test kit according to any one of [21] to [23], in which the glass-made member is a filter for specimen filtration including glass fibers.
[25] The test kit according to any one of [21] to [24], in which the substance having an isoelectric point of 10.0 or more is derived from a respiratory infection virus.
[26] The test kit according to [25], in which the respiratory infection virus is a coronavirus.
[27] The test kit according to [26], in which the coronavirus is SARS-CoV-2.
[28] The test kit according to any one of [21] to [27], further including a specimen-diluting liquid.
[29] The test kit according to [28], in which the cationic substance is contained in the specimen-diluting liquid.
[30] The test kit according to any one of [21] to [29], in which the cationic substance is at least one selected from the group consisting of a metal salt, a cationic polymer, a cationic peptide, and a cationic surfactant.
[31] A qualitative or quantitative test method of a substance to be detected contained in a specimen, using the test kit according to any one of [21] to [30].
[32] A qualitative or quantitative test method to detect a substance to be detected contained in a specimen, the test method including
   a preparation step of preparing a test sample from the specimen, and
   a test step of testing at least part of the test sample with a reagent to detect each of a first substance to be detected and a second substance to be detected,
   in which the first substance to be detected has an isoelectric point of 10.0 or more, the substance having an isoelectric point of 10.0 or more is derived from a respiratory infection virus, and the respiratory infection virus is a coronavirus, and
   in any one or both of the preparation step and the test step, the first substance to be detected is brought into contact with any one or both of glass-made member and nitrocellulose-made member in a presence of at least one cationic substance selected from the group consisting of a metal salt which is sodium citrate or magnesium sulfate, a cationic polymer which is a cationic polymer having hexadimethrine bromide or 2-methacryloyloxyethyl phosphorylcholine as a constitutional unit, a cationic peptide which is cocodimonium hydroxypropyl hydrolyzed keratin, and a cationic surfactant which is an alkylamine salt surfactant or a quaternary ammonium salt surfactant.
[33] The test method according to [32], in which the reagent includes a sample supply part and a labeled reagent holding part, and any one or both of the sample supply part and the labeled reagent holding part include the glass-made member.
[34] A qualitative or quantitative test method to detect a substance to be detected contained in a specimen, the test method including
   a preparation step of preparing a test sample from the specimen,
   a first test step of testing at least part of the test sample with a first reagent to detect each of a first substance to be detected and a second substance to be detected, and
   a second test step of testing part of the test sample with a second reagent to detect the second substance to be detected,
   in which the first substance to be detected has an isoelectric point of 10.0 or more, the substance having an isoelectric point of 10.0 or more is derived from a respiratory infection virus, and the respiratory infection virus is a coronavirus, and
   in any one or both of the preparation step and the first test step, the first substance to be detected is brought into contact with any one or both of glass-made member and nitrocellulose-made member in a presence of at least one cationic substance selected from the group consisting of a metal salt which is sodium citrate or magnesium sulfate, a cationic polymer which is a cationic polymer having hexadimethrine bromide or 2-methacryloyloxyethyl phosphorylcholine as a constitutional unit, a cationic peptide which is cocodimonium hydroxypropyl hydrolyzed keratin, and a cationic surfactant which is an alkylamine salt surfactant or a quaternary ammonium salt surfactant.
[35] The test method according to [34], in which the first reagent includes a sample supply part and a labeled reagent holding part, and any one or both of the sample supply part and the labeled reagent holding part include the glass-made member.
[36] The test method according to any one of [32] to [35], in which the preparation step includes a step of suspending the specimen in a specimen-diluting liquid.
[37] The test method according to [36], in which the cationic substance is contained in the specimen-diluting liquid.
[38] The test method according to any one of [32] to [35], in which the preparation step includes a step of filtering the test sample with a filter.
[39] The test method according to any one of [32] to [35], in which the glass-made member is a filter for specimen filtration including glass fibers.
[40] The test method according to any one of [32] to [35], in which the coronavirus is SARS-CoV-2.
[41] A reagent including
   a first labeled reagent in which a first binding substance capable of binding to a first substance to be detected is labeled with a labeling substance,
   a second labeled reagent in which a second binding substance capable of binding to a second substance to be detected is labeled with a labeling substance,
   a membrane on which a first detection part in which a first solid phase reagent capable of binding to the first substance to be detected is immobilized and a second detection part in which a second solid phase reagent capable of binding to the second substance to be detected is immobilized are arranged, and
   any one or both of a glass-made member and a nitrocellulose-made member arranged upstream of the first detection part in a development direction of a development liquid containing a specimen,
   in which the first substance to be detected has an isoelectric point of 10.0 or more and derived from a coronavirus, which is a respiratory infection virus, and
   the development liquid brought into contact with any one or both of the glass-made member and the nitrocellulose-made member contains at least one cationic substance selected from the group consisting of a metal salt which is sodium citrate or magnesium sulfate, a cationic polymer which is a cationic polymer having hexadimethrine bromide or 2-methacryloyloxyethyl phosphorylcholine as a constitutional unit, a cationic peptide which is cocodimonium hydroxypropyl hydrolyzed keratin, and a cationic surfactant which is an alkylamine salt surfactant or a quaternary ammonium salt surfactant, and the cationic substance is held in a constituent material of the reagent.
[42] The reagent according to [41], in which the reagent includes a sample supply part and a labeled reagent holding part, and any one or both of the sample supply part and the labeled reagent holding part include the glass-made member.
[43] The reagent according to [41] or [42], in which the glass-made member is a filter for specimen filtration including glass fibers.
[44] The reagent according to any one of [41] to [44], in which the coronavirus is SARS-CoV-2.
[45] A qualitative or quantitative test method of a substance to be detected contained in a specimen using the reagent according to any one of [41] to [44].
[46] A test kit including a reagent and a specimen-diluting liquid containing a cationic substance,
   in which the reagent includes a first labeled reagent in which a first binding substance capable of binding to a first substance to be detected is labeled with a labeling substance, a second labeled reagent in which a second binding substance capable of binding to a second substance to be detected is labeled with a labeling substance, and a membrane on which a first detection part in which a first solid phase reagent capable of binding to the first substance to be detected is immobilized and a second detection part in which a second solid phase reagent capable of binding to the second substance to be detected is immobilized are arranged,
   the test kit includes any one or both of a glass-made member and a nitrocellulose-made member arranged upstream of the first detection part in a development direction of a development liquid containing a specimen,
   the first substance to be detected has an isoelectric point of 10.0 or more and derived from a coronavirus which is a respiratory infection virus,
   the development liquid brought into contact with any one or both of the glass-made member and the nitrocellulose-made member contains a cationic substance, and
   the cationic substance contains at least one selected from the group consisting of a metal salt which is sodium citrate or magnesium sulfate, a cationic polymer which is a cationic polymer having hexadimethrine bromide or 2-methacryloyloxyethyl phosphorylcholine as a constitutional unit, a cationic peptide which is cocodimonium hydroxypropyl hydrolyzed keratin, and a cationic surfactant which is an alkylamine salt surfactant or a quaternary ammonium salt surfactant.
[47] A test kit including a specimen filtration filter, a specimen-diluting liquid containing a cationic substance, and a reagent
   in which the reagent includes a first labeled reagent in which a first binding substance capable of binding to a first substance to be detected is labeled with a labeling substance, a second labeled reagent in which a second binding substance capable of binding to a second substance to be detected is labeled with a labeling substance, and a membrane on which a first detection part in which a first solid phase reagent capable of binding to the first substance to be detected is immobilized and a second detection part in which a second solid phase reagent capable of binding to the second substance to be detected is immobilized are arranged,
   the test kit includes any one or both of a glass-made member and a nitrocellulose-made member arranged upstream of the first detection part in a development direction of a development liquid containing any one or both of a specimen of the specimen filtration filter and a specimen of the reagent,
   the first substance to be detected has an isoelectric point of 10.0 or more and derived from a coronavirus, which is a respiratory infection virus,
   the development liquid brought into contact with any one or both of the glass-made member and the nitrocellulose-made member contains a cationic substance, and
   the cationic substance contains at least one selected from the group consisting of a metal salt which is sodium citrate or magnesium sulfate, a cationic polymer which is a cationic polymer having hexadimethrine bromide or 2-methacryloyloxyethyl phosphorylcholine as a constitutional unit, a cationic peptide which is cocodimonium hydroxypropyl hydrolyzed keratin, and a cationic surfactant which is an alkylamine salt surfactant or a quaternary ammonium salt surfactant.
[48] The test kit according to [46] or [47], in which the reagent includes a sample supply part and a labeled reagent holding part, and any one or both of the sample supply part and the labeled reagent holding part include the glass-made member.
[49] The test kit according to any one of [46] to [48], in which the glass-made member is a filter for specimen filtration including glass fibers.
[50] The test kit according to any one of [46] to [49], in which the coronavirus is SARS-CoV-2.
[51] A qualitative or quantitative test method of a substance to be detected contained in a specimen, using the test kit according to any one of [46] to [50]. Advantageous Effects of Invention

According to the present invention, a test method is provided in which two or more substances to be detected including a substance having an isoelectric point of 10.0 or more can be detected with high sensitivity using the same specimen while suppressing a decrease in measurement sensitivity.

### Brief Description of Drawings

[FIG. 1] This is a schematic view of an example of an embodiment of a reagent.
[FIG. 2] This is a schematic diagram of a configuration of a reagent used in Examples.

### Description of Embodiments

The present inventors have conducted extensive studies to achieve the above-described object, and have found that two or more substances to be detected including a substance having an isoelectric point of 10.0 or more can be tested with high sensitivity by bringing a substance having an isoelectric point of 10.0 or more into contact with any one or both of a glass-made member or a nitrocellulose-made member in the presence of a cationic substance.

### [Substance to be detected]

Examples of the substance to be detected according to the embodiment of the present invention include pathogens such as viruses and physiologically active substances such as proteins. Examples of the substance to be detected according to the embodiment of the present invention include markers related to various diseases, that is, infection-related markers such as respiratory infection virus, norovirus, human immunodeficiency virus (HIV), hepatitis B virus (HBV), hepatitis C virus (HCV), mycoplasma, toxoplasma, chlamydia, syphilis, staphylococcus aureus, and Escherichia coli; myocardial markers such as CK-MB, H-FABP, troponin, and myoglobin; coagulation/fibrinolysis markers such as fibrin degradation products (for example, D-dimer), soluble fibrin, TAT (thrombin-antithrombin complex), and PIC (plasmin-plasmin inhibitor complex); circulation-related markers such as oxidized LDL and BNP (brain natriuretic peptide); metabolism-related markers such as adiponectin; tumor markers such as CEA (cancer embryonic antigen), AFP (α-fetoprotein), CA19-9, CA125, and PSA (prostate-specific antigen); and inflammation-related markers such as CRP (C-reactive protein), IgA, IgG, and IgM.

As the substance to be detected according to the embodiment of the present invention, a respiratory infection virus is preferable. In the present specification, the respiratory infection virus is a virus that causes a respiratory system disease. The respiratory infection virus according to the embodiment of the present invention is not particularly limited as long as it is a virus that can be detected by an antigen-antibody reaction, and examples thereof include coronaviruses such as SARS-CoV, MERS-CoV, SARS-CoV-2, HCoV-229E, HCoV-OC43, HCoV-NL63, and HCoV-HKU1; influenza viruses such as an influenza A virus, an influenza B virus, and an influenza C virus;, parainfluenza viruses such as a parainfluenza virus 1 a parainfluenza virus 2, and a parainfluenza virus 3; RS viruses such as an RS virus A type and an RS virus B type; an adenovirus; a rhinovirus; a metapneumovirus; a cytomegalovirus; a bocavirus; and the like. Among these, it is preferable that the substance to be detected be SARS-CoV-2, an influenza virus such as an influenza A virus or an influenza B virus, an RS virus such as an RS virus A type or an RS virus B type, and/or an adenovirus. **In** the present specification, the term "respiratory organ" is a general term for an organ related to respiration, and refers to an organ from the nasal vestibule to an alveolus (the lungs) through the nasal cavity, pharynx, larynx, trachea, bronchus, and bronchioles.

**In** the test method according to the embodiment of the present invention, at least the first substance to be detected and the second substance to be detected are tested qualitatively or quantitatively.

The first substance to be detected has an isoelectric point of 10.0 or more.

The substance having an isoelectric point of 10.0 or more is not particularly limited as long as it can be detected by an antigen-antibody reaction, and examples thereof include an exogenous substance (virus, bacteria, administered drug, and the like), a protein caused by the exogenous substance (metabolite of the exogenous substance, secretion of bacteria, and the like), and an endogenous substance (antibody produced in the body, hormone, and the like). The substance having an isoelectric point of 10.0 or more includes a protein, a peptide, or a nucleic acid, and a protein having an isoelectric point of 10.0 or more is preferable.

The first substance to be detected may be a protein having an isoelectric point of 10.0 or more, 10.1 or more, 10.2 or more, 10.3 or more, 10.4 or more, or 10.5 or more.

The isoelectric point is a pH at which an average charge of the entire compound after ionization is 0 in a compound having both a functional group which becomes an anion and a functional group which becomes a cation.

The substance having an isoelectric point of 10.0 or more in the test method according to the embodiment of the present invention is preferably a protein derived from a virus, more preferably a protein derived from a respiratory infection virus, still more preferably a protein derived from a coronavirus, and most preferably a protein derived from SARS-CoV-2. The substance having an isoelectric point of 10.0 or more may be a virus itself constituted of a plurality of proteins, or may be part of a protein constituting a virus and a fragment thereof. The substance having an isoelectric point of 10.0 or more is preferably a nucleocapsid protein of a coronavirus, and more preferably a nucleocapsid protein of SARS-CoV-2.

### [Test method]

### <First embodiment>

A first embodiment of the test method according to the present invention includes a preparation step of preparing a test sample from a specimen, and a test step of testing at least part of the test sample with a reagent to detect each of the first substance to be detected and the second substance to be detected.

As described above, the first substance to be detected has an isoelectric point of 10.0 or more.

In the test method according to the first embodiment, in any one or both of the preparation step and the test step, the first substance to be detected is brought into contact with any one or both of a glass-made member or a nitrocellulose-made member in the presence of the cationic substance.

The preparation step may include, for example, a step of separating a specimen, a step of suspending a specimen in a specimen-diluting liquid, a step of diluting a specimen with a specimen-diluting liquid, and a step of filtering a test sample with a filter. It is preferable that the specimen-diluting liquid contain a cationic substance. The cationic substance will be described later.

In the detection method according to the first embodiment, a reagent configured to be able to test two or more substances to be detected with a single reagent is used such that a plurality of types of substances to be detected can be tested in one test.

In the test method according to the first embodiment, examples of the specimen that may contain the substance to be detected principally include a biological sample derived from a living thing, an extracted liquid obtained by extracting the substance to be detected from the biological sample, and the like. A food specimen represented by a liquid beverage, a semi-solid food, a solid food, and the like, a sampling specimen from the natural world such as soil, a river, and sea water, and a specimen wiped from a production line or a clean room in a factory can be also used as the sample that may contain the substance to be detected.

As the specimen that may contain the substance to be detected, a biological sample is preferable.

Examples of the biological sample include blood, serum, blood plasma, urine, tear, ear discharge, prostate fluid, and respiratory tract secretion, but the biological sample is not limited thereto. The biological sample is preferably at least one selected from the group consisting of blood, serum, blood plasma, urine, tear, ear discharge, prostate fluid, and respiratory tract secretion, and more preferably a respiratory tract secretion. The respiratory tract secretion means a body fluid secreted within a tissue or on a surface of a respiratory tract. Examples of the respiratory tract secretion include body fluids secreted in the nostrils, nasal cavity, pharynx, nasopharynx, oral cavity, and the like, but the respiratory tract secretion is not limited thereto. As the respiratory tract secretion, a nasopharyngeal swab solution, a nasal swab solution, saliva, or sputum is preferable.

The target from which the biological sample is collected includes a human or a mammal (for example, a monkey, a dog, or a cat), but is not limited thereto. A human is preferable as the target. The person who collects or prepares the biological sample may be the same person or may be a different person from the person who performs the test method according to the first embodiment. In addition, the biological sample used in the test method according to the first embodiment may be collected or prepared at the time of performing the test method according to the embodiment of the present invention, or may be collected or prepared in advance and stored.

The specimen-diluting liquid is a solution for suspending or diluting the biological sample. The specimen-diluting liquid preferably contains a cationic substance described later. In a case where the specimen-diluting liquid contains a cationic substance, it is preferable that the specimen-diluting liquid contain the cationic substance at the time of sale, but in a case where the test kit includes the specimen-diluting liquid, the cationic substance may be contained separately from the specimen-diluting liquid. In this case, it is preferable that the person who performs the preparation step mix the cationic substance with the specimen-diluting liquid.

The specimen-diluting liquid preferably contains a buffer solution. The "buffer solution" means a solution having a pH buffering action. Examples of the buffer solution used in the test method according to the first embodiment include a conventionally known buffer solution such as PBS (phosphate-buffered saline), MES (2-(N-morpholino)ethanesulfonic acid), PIPES (piperazine-N,N' -bis(2-ethanesulfonic acid)), ACES (N-(2-acetamido)-2-aminoethanesulfonic acid), ADA (N-(2-acetamido)iminodiacetic acid), Bis-Tris (2,2-bis(hydroxyethyl)-(iminotris)-(hydroxymethyl)-methane), Tris (tris(hydroxymethyl)aminomethane), MOPS (3-morpholinopropanesulfonic acid), HEPES (4-(2-hydroxyethyl)-1-piperazineethanesulfonic acid), citrate buffer solution, glycine buffer solution, borate buffer solution, and phosphate buffer solution, but the buffer solution is not limited thereto. As the buffer solution used in the test method according to the first embodiment, at least one selected from the group consisting of PBS, MES, PIPES, ACES, ADA, Bis-Tris (2,2-bis(hydroxyethyl)-(iminotris)-(hydroxymethyl)-methane), qTris (tris(hydroxymethyl)aminomethane), MOPS, HEPES, a citrate buffer solution, a glycine buffer solution, a borate buffer solution, and a phosphate buffer solution is preferable, and a phosphate buffer solution is more preferable.

The step of separating the specimen is a step of separating the part to be tested from the collected specimen.

The step of suspending the specimen in the specimen-diluting liquid is exemplified below.

In a case where the specimen is a swab solution collected from the nasopharynx, the nasal cavity, or the like with a swab, the specimen can be suspended in the specimen-diluting liquid by inserting the swab into the specimen-diluting liquid and rotating the swab. In a case where the container that holds the specimen-diluting liquid is formed of a plastic material, the specimen can also be suspended in the specimen-diluting liquid by crushing the swab with which the specimen has been collected, together with the container. In a case where the specimen is a liquid such as a nasal aspirate, saliva, or blood, the specimen can be taken in a necessary amount using a pipette or the like and suspended in the specimen-diluting liquid, or the specimen can be also suspended in the specimen-diluting liquid by inserting a swab immersed in the specimen into the specimen-diluting liquid, as in the case of the swab solution.

The step of diluting the specimen with the specimen-diluting liquid is a step of diluting the specimen with the specimen-diluting liquid in advance in a case where it is assumed that the specimen contains a substance to be detected exceeding the measurement upper limit of the reagent, or the like.

The step of filtering through a filter is a step of removing a solid substance which may be contained in the specimen or the test sample by using a filter. For example, the step of filtering can be performed as follows.

The specimen filtration filter consisting of a filter and a support for holding the filter is inserted into a container holding the specimen or a specimen-diluting liquid containing the specimen. In this case, the specimen inflow side of the specimen filtration filter and the opening side of the container are formed such that one is closely attached to and fitted to the other. The specimen or the specimen-diluting liquid containing the specimen filtered from the distal end side of the specimen filtration filter opposite the specimen inflow side is obtained by changing the holding manner of the container into which the specimen filtration filter is inserted such that the specimen filtration filter is located below and crushing the container. At this time, the obtained filtrate can be directly added dropwise to the reagent, or the filtrate can be held in another container.

The test step in the test method according to the first embodiment includes a step of bringing the reagent into contact with the test sample and a step of reading the test result from the reagent after a predetermined time has elapsed.

In the test method according to the first embodiment, any one or both of the preparation step or the test step includes a step of bringing the first substance, which is a substance to be detected having an isoelectric point of 10.0 or more, into contact with any one or both of the glass-made member or the nitrocellulose-made member in the presence of the cationic substance. It is considered that, in a case of coexisting the cationic substance when the substance having an isoelectric point of 10.0 or more, which is positively charged in the test sample, is brought into contact with the glass-made member or the nitrocellulose-made member which is negatively charged, the substance having an isoelectric point of 10.0 or more is suppressed from being adsorbed on the glass-made member or the nitrocellulose-made member. The above mechanism does not limit the scope of the present invention.

The order in which the specimen containing the cationic substance and the substance to be detected are brought into contact with any one or both of the glass-made member and the nitrocellulose-made member is not particularly limited as long as the cationic substance coexists when the specimen containing the substance to be detected is brought into contact with the glass-made member or the nitrocellulose-made member. That is, the cationic substance can be brought into contact with any one or both of the glass-made member or the nitrocellulose-made member before the specimen containing the substance to be detected, or the cationic substance can be brought into contact with any one or both of the glass-made member or the nitrocellulose-made member at the same time as the specimen containing the substance to be detected. It is preferable that the specimen containing the substance to be detected be brought into contact with any one or both of the glass-made member and the nitrocellulose-made member after mixing the specimen containing the substance to be detected with the cationic substance, or at the same time as the mixing, and it is more preferable that the specimen containing the substance to be detected be brought into contact with the glass-made member and/or the nitrocellulose-made member after mixing the specimen containing the substance to be detected with the cationic substance.

The cationic substance may be contained in the specimen-diluting liquid, or may be held in the constituent material of the test kit, that is, in the specimen filtration filter, the sample pad, the conjugate pad, the antibody-immobilized membrane, or any site located between the sample supply part and the membrane carrier and different from the sample pad and the conjugate pad. **In** a case where the cationic substance is held in the constituent material of the test kit, the cationic substance is eluted or flows into the specimen or the test sample when the specimen or the test sample is brought into contact with the constituent material, resulting in the development liquid containing the cationic substance.

The cationic substance refers to a substance which has a cationic functional group and has a positive charge in a case of being in contact with water. In a case where the cationic substance has both the cationic functional group and the anionic functional group, the cationic substance may have a positive charge in the entire molecule.

Examples of the cationic functional group include amino groups such as a primary amino group, a secondary amino group, and a tertiary amino group; onium bases such as a quaternary ammonium group and a phosphonium group; amino acid residues such as an arginyl group, a lysyl group, a histidyl group, and a guanidyl group; a heterocyclic group such as an imidazole group; and the like. Examples of the cationic substance can include at least one selected from the group consisting of a metal salt, a cationic polymer, a cationic surfactant, and a cationic peptide. The cationic substance is preferably a metal salt, a cationic polymer, or a cationic surfactant and more preferably a cationic polymer, includes still more preferably both a cationic polymer and a metal salt, and includes even still more preferably both a cationic polymer and sodium chloride.

As the metal salt, a metal salt ionized in an aqueous solution to generate a metal ion can be used without any particular limitation. Even in a case where the above-described cationic functional group is not included, in a metal salt ionized in a case of being brought into contact with water, a cationic metal ion is generated. Examples of the metal ion include an alkali metal ion, an alkaline earth metal ion, and other metal ions, and an alkali metal ion or an alkaline earth metal ion is preferable. Examples of the alkali metal ion include a lithium ion, a sodium ion, and a potassium ion, and the like, examples of the alkaline earth metal ion include a magnesium ion, a calcium ion, and the like, and examples of the other metal ions include a copper ion and the like. The alkali metal ion is preferably a sodium ion, and the alkaline earth metal ion is preferably a magnesium ion. The alkali metal salt is preferably sodium chloride or sodium citrate, and the alkaline earth metal salt is preferably magnesium sulfate.

**In** the present invention, the "cationic polymer" means a polymer having a cationic functional group in the side chain as the side chain. The cationic functional group as the side chain is preferably a quaternary ammonium group. The cationic polymer is preferably a cationic polymer having a bromide hexadimethrine (CAS RN 28728-55-4) or 2-methacryloyloxyethyl phosphorylcholine as a constitutional unit, in which an aqueous solution obtained by dissolving the cationic polymer in water at 0.5% by mass has a dynamic viscosity of 20 to 60 m²/s at 25°C, an aqueous solution obtained by dissolving the cationic polymer in water at 0.1% by mass has a surface tension of 70 × 10⁻³ to 80 × 10⁻³ N/m at 25°C, and a mass-average molecular weight (Mw) is 50 to 600 kDa. The cationic polymer having 2-methacryloyloxyethyl phosphorylcholine as a constitutional unit is commercially available as Lipidure (registered trademark)-BL502.

The dynamic viscosity η [m²/s] of the aqueous solution of the cationic polymer in an aqueous solution at a concentration of 0.5% by mass at 25°C can be obtained by dividing a viscosity µ [Pa·s] obtained by measuring an aqueous solution obtained by dissolving the cationic polymer in water at a concentration of 0.5% by mass at 25°C with a Ubbelohde viscometer by a density ρ [kg/m³] of the aqueous solution at 25°C.

The surface tension γ [mN/m] of the aqueous solution of the cationic polymer at a concentration of 0.1% by mass at 25° C can be obtained by measuring an aqueous solution obtained by dissolving the cationic polymer in water at a concentration of 0.1% by mass, by a Du Nouy ring method, a Wilhelmy plate method, or a drop-weight method at 25° C.

The mass-average molecular weight (Mw) of the cationic polymer can be determined by a gel permeation chromatography (GPC) method. Polyethylene glycol is used as the standard substance.

In the present invention, the term "cationic surfactant" means a surfactant in which a portion of the surfactant to which a hydrophobic group is bonded is positively charged in a case where the surfactant is dissolved in an aqueous solution. Examples of the cationic surfactant include an alkylamine salt surfactant and a quaternary ammonium salt surfactant.

Examples of the alkylamine salt surfactant include a salt of an amine including 1 to 3 alkyl groups having 8 to 22 carbon atoms, such as monododecylamine, monooctadecylamine, dioctadecylamine, and trioctadecylamine, and an inorganic acid such as hydrochloric acid and sulfuric acid or a lower carboxylic acid such as acetic acid, lactic acid, and citric acid, and the like. Specific examples thereof include stearylamine acetate [CAS number: 2190-04-7, product name: ACETAMINE 86, manufactured by Kao Corporation], and the like.

Examples of the quaternary ammonium salt surfactant include a salt of ammonium containing 1 to 3 alkyl groups having 8 to 22 carbon atoms, such as dodecyltrimethylammonium, octadecyltrimethylammonium, hexadecyltrimethylammonium, didecyldimethylammonium, and benzylmethyltetradecylammonium, and chlorine, bromine, or the like, and the like. Specific examples thereof include lauryltrimethylammonium chloride [CAS number: 112-00-5, product name: QUARTAMINE (registered trademark) 24P, manufactured by Kao Corporation] and dodecyltrimethylammonium bromide [CAS number: 1119-94-4, manufactured by Tokyo Chemical Industry Co., Ltd.].

The cationic peptide means a peptide which exhibits cationicity in a case of being dissolved or dispersed in water. The cationic peptide may be a peptide containing many basic amino acid residues (arginine (Arg), lysine (Lys), or histidine (His)) as an amino acid residue constituting the peptide. The cationic peptide is not particularly limited as long as it exhibits cationicity in a case of being dissolved or dispersed in water, and for example, polyarginine, polylysine, polyhistidine, or the like, which is a polymer of the basic amino acid residue, can be used. As the cationic peptide, it is preferable to use cocodimonium hydroxypropyl hydrolyzed keratin (CAS RN 68915-25-3). The cocodimonium hydroxypropyl hydrolyzed keratin is commercially available from SEIWA KASEI CO., LTD. as a trade name Promois (registered trademark) WK-HCAQ.

The concentration of the cationic substance can be appropriately modified depending on the type of substance to be detected, the type of sample, and the type of immunoassay method. The concentration of the cationic substance indicates a concentration of the cationic substance in a case where the cationic substance is contained in the specimen-diluting liquid or a concentration of the cationic substance in a case where the cationic substance is contained in the development liquid. For example, in a case where the cationic substance is a metal salt, the concentration of the cationic substance is 0.1 to 1,000 mM, 1 to 750 mM, or 2 to 750 mM. In a case where the cationic substance is an alkali metal salt, the concentration of the cationic substance is 0.1 to 1,000 mM, 1 to 750 mM, 2 to 750 mM, 50 to 750 mM, or 200 to 750 mM. In a case where the cationic substance is an alkaline earth metal salt, the concentration of the cationic substance is 0.1 to 1,000 mM, 1 to 500 mM, 1 to 250 mM, or 1 to 100 mM. In addition, in a case where the cationic substance is the cationic polymer, the cationic surfactant, or the cationic peptide, the concentration of the cationic substance is 0.0001% to 20% by mass, 0.0005% to 10% by mass, 0.001% to 5% by mass, 0.01% to 1% by mass, or 0.01% to 0.5% by mass. Even in a case where the specimen-diluting liquid or the development liquid contains a plurality of types of cationic substances, the concentration of each component can be adopted in the above-described range.

The glass-made member contains glass, and a composite member of glass and another material is also included in the glass-made member. "Glass" refers to glass in which silicon dioxide (SiO₂) is used as a constituent component. Glass in which silicon dioxide (SiO₂) is used as a constituent component has a network structure based on a tetrahedron of SiO₄ on a low-temperature side with respect to a glass transition point. In this network structure, the oxygen atom bonded to the silicon atom is negatively charged.

Examples of the glass-made member include a sample pad of the reagent, a conjugate pad, or any pad (third pad) located between a sample supply part and a membrane, which is different from the sample pad and the conjugate pad, and a specimen filtration filter.

The nitrocellulose-made member contains nitrocellulose, and a composite material of nitrocellulose and another material is also included in the nitrocellulose-made member. Nitrocellulose is also negatively charged in the same manner as the glass fibers. Examples of the nitrocellulose material include a nitrocellulose membrane and a specimen filtration filter.

As the reagent used in the test method according to the first embodiment, a reagent for performing a test by developing a specimen in the reagent by using a capillary action or the like is preferable. Examples of the reagent used in the test method according to the first embodiment include an immunochromatography test strip, a nucleic acid chromatography test strip, and a microfluidic device. Examples of the immunochromatography test strip and the nucleic acid chromatography test strip include a test strip in which a sample pad, a membrane, and an absorption pad are arranged in this order from the upstream to the downstream in the flow direction of the test sample. Each of the members is arranged such that the upstream and downstream pads and at least part thereof overlap with each other. On the sample pad, a sample supply part and a labeled reagent holding part can be formed. A detection part is arranged on the membrane. Examples of the microfluidic device include a device in which a sample supply part, a labeled reagent holding part, and a detection part are connected by a micro flow channel. The microfluidic device may be provided with a pump for control of a flow rate or the like, or a connecting portion for connection to the pump.

In the reagent used in the test method according to the first embodiment, at least one of the sample supply part or the labeled reagent holding part preferably includes a glass-made member. However, in a case where the sample is filtered with a specimen filtration filter including a glass-made member before the sample is supplied to the sample supply part, neither the sample supply part nor the labeled reagent holding part may include a glass-made member.

The sample supply part is a portion that supplies the test sample to the reagent. The sample supply part of the immunochromatography test strip and the nucleic acid chromatography test strip can be formed on part of the sample pad. Examples of a material constituting the sample pad include a pad consisting of a material such as paper, a cellulose mixture, nitrocellulose, polyester, an acrylonitrile copolymer, glass fibers, and rayon. The sample pad is preferably a pad consisting of glass fibers or polyester, and more preferably a pad consisting of glass fibers. The sample supply part of the microfluidic device can be formed in the opening portion of the micro flow channel.

The labeled reagent holding part holds a labeled reagent described later. The labeled reagent holding part of the immunochromatography test strip and the nucleic acid chromatography test strip can be arranged on part of the sample pad, or can be formed as a conjugate pad separate from the sample pad. In a case where the labeled reagent holding part is formed as the conjugate pad, the labeled reagent may be arranged on the entire conjugate pad or may be arranged on part of the conjugate pad. The labeled reagent holding part can be formed on a membrane described later, without disposing the conjugate pad. The labeled reagent holding part is preferably arranged in a line shape on part of the sample pad or the conjugate pad. The labeled reagent holding part of the microfluidic device can be formed between the opening portion of the micro flow channel and the detection part.

The labeled reagent is obtained by labeling a binding substance capable of binding to the substance to be detected with a labeling substance. Examples of a method of labeling a binding substance capable of binding to the substance to be detected with a labeling substance include physical adsorption, chemical bonding, and the like.

Examples of the labeling substance include a metal complex, an enzyme, insoluble particles, a fluorescent substance, a chemiluminescent substance, biotin, avidin, a radioactive isotope, gold colloidal particles, and a colored latex. It is preferable to use gold colloidal particles as the labeling substance.

The binding substance capable of binding to the substance to be detected means a substance that binds to the substance to be detected by an antigen-antibody reaction or the like. **In** a case where the substance to be detected is a protein, the binding substance is preferably an antibody or a fragment of an antibody. The antibody may be any of a monoclonal antibody or a polyclonal antibody, and both of which can be produced according to a known method. The binding substance capable of binding to the substance to be detected according to the embodiment of the present invention is preferably a monoclonal antibody.

The detection part is a portion that detects the substance to be detected. The detection part of the immunochromatography test strip and the nucleic acid chromatography test strip is formed by immobilizing a binding substance capable of binding to the substance to be detected on a membrane. The detection parts of the number corresponding to the number of the substances to be detected are arranged on the membrane. **In** a case where there are two types of substances to be detected, the first detection part and the second detection part are arranged, and either the first detection part or the second detection part may be upstream. For example, in a case of testing a first substance to be detected and a second substance to be detected, a first detection part in which a first solid phase reagent capable of binding to the first substance to be detected is immobilized and a second detection part in which a second solid phase reagent capable of binding to the second substance to be detected is immobilized are arranged on the membrane. In this case, either the first detection part or the second detection part may be arranged on the upstream side in the test sample development direction. A control part for confirming an appropriate progress of the test may be arranged on the membrane. The membrane is preferably formed of a nitrocellulose-made member. A detection part of the microfluidic device is formed by immobilizing a binding substance capable of binding to a substance to be detected on part of the micro flow channel.

The absorption pad is a member that absorbs the test sample that has moved or passed through the membrane. As the absorption pad, a material made of cellulose or the like can be used.

### <Second embodiment>

A second embodiment of the test method according to the present invention includes a preparation step of preparing a test sample from a specimen, a first test step of testing at least part of the test sample with a first reagent for detecting a first substance to be detected, and a second test step of testing part of the test sample with a second reagent for detecting a second substance to be detected.

As described above, the first substance to be detected has an isoelectric point of 10.0 or more.

In the test method according to the second embodiment, in any one or both of the preparation step and the first test step, the first substance to be detected is brought into contact with any one or both of a glass-made member or a nitrocellulose-made member in the presence of the cationic substance.

In the detection method according to the second embodiment, the step of testing with the reagent used in the first test step and the second test step includes a step of bringing the reagent into contact with the test sample, and a step of reading the test result from the reagent after a predetermined time has elapsed.

In the detection method according to the second embodiment, any one or both of the preparation step or the first test step includes a step of bringing the first substance to be detected, which is a substance having an isoelectric point of 10.0 or more, into contact with any one or both of the glass-made member or the nitrocellulose-made member in the presence of the cationic substance. It is considered that, in a case of coexisting the cationic substance when the substance having an isoelectric point of 10.0 or more, which is positively charged in the test sample, is brought into contact with the glass-made member or the nitrocellulose-made member which is negatively charged, the substance having an isoelectric point of 10.0 or more is suppressed from being adsorbed on the glass-made member or the nitrocellulose-made member. The above mechanism does not limit the scope of the present invention.

Since the detection method according to the second embodiment is the same as the detection method according to the first embodiment, except that each of the substances to be detected is tested using an individual reagent, the description thereof will be omitted.

### [Test kit and reagent] <First embodiment>

A test kit and a reagent for performing the test method according to the first embodiment of the present invention will be described.

### (Reagent)

The reagent according to the first embodiment will be described with reference to an immunochromatography test strip as an example. Hereinafter, the immunochromatography test strip may be simply referred to as a test strip.

The test strip according to the first embodiment includes a glass-made member brought into contact with a test sample containing a protein having an isoelectric point of 10.0 or more. The glass-made member is preferably at least one selected from the group consisting of a filter for specimen filtration, a sample pad, a conjugate pad, and a third pad.

The test strip according to the first embodiment contains a cationic substance brought into contact with a test sample containing a protein having an isoelectric point of 10.0 or more. The cationic substance is contained in at least one selected from the group consisting of a specimen-diluting liquid, a filter for specimen filtration, and a test strip, is preferably contained in at least one selected from the group consisting of a specimen-diluting liquid, a filter for specimen filtration, a sample pad, a conjugate pad, and a third pad, and is more preferably contained in a specimen-diluting liquid. However, the cationic substance is contained in a portion where the glass-made material and the sample are to be brought into contact with each other or an upstream side of the portion.

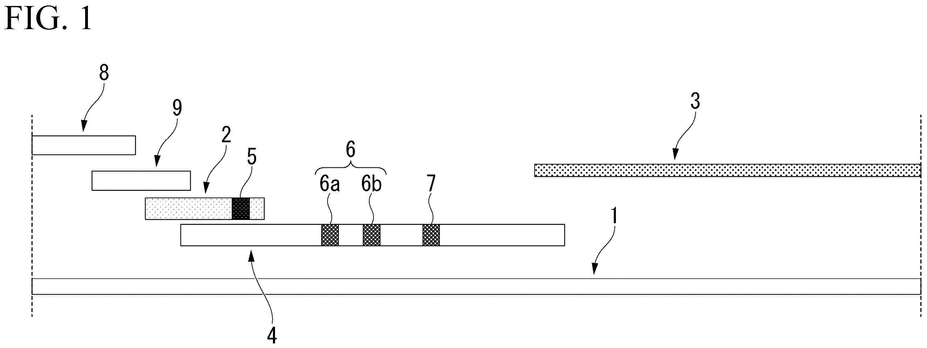

FIG. 1 shows a configuration of one embodiment of a test strip according to the first embodiment. In the test strip shown in FIG. 1, a sample pad 8, a third pad 9, a conjugate pad 2, an insoluble membrane carrier 4, and an absorption pad 3 are arranged in this order from the upstream to the downstream in the flow direction of the test sample. The pads are arranged such that at least part of each pad and the upper and lower pads thereof overlap each other. A test line (detection part) 6 and a control line 7 are arranged on the insoluble membrane carrier 4. The test line (detection part) 6 includes a first test line (first detection part) 6a to detect the first substance to be detected and a second test line (second detection part) 6b to detect the second substance to be detected. The number of the test lines can be increased according to the number of the substances to be detected. It is preferable that a cationic substance be added to at least one of the sample pad 8, the third pad 9, or the conjugate pad 2.

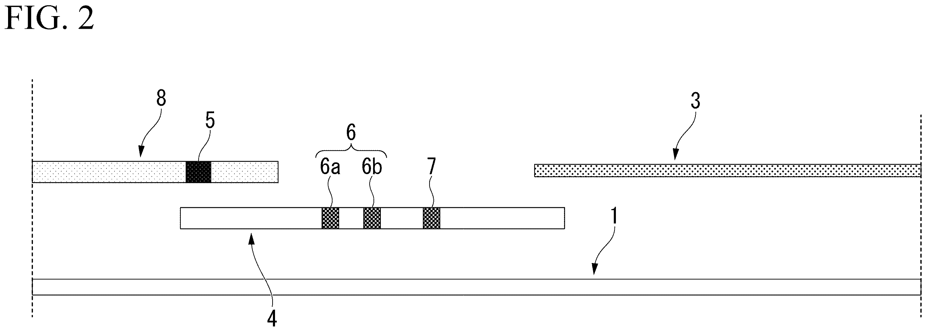

FIG. 2 shows a configuration of a modification example of the test strip according to the first embodiment. The test strip shown in FIG. 2 is obtained by omitting the conjugate pad 2 and the third pad 9 from the basic form of the test strip of the first embodiment shown in FIG. 1 and arranging the labeled reagent holding part 5 on the sample pad 8.

The test sample, the specimen-diluting liquid, and the cationic substance are the same as those in the above-described test method.

The test strip according to the first embodiment is configured to be able to test two or more substances with a single immunochromatography test strip such that a plurality of types of substances can be tested in one test. Specifically, the conjugate pad contains an antibody corresponding to each of the plurality of substances to be detected, and an antibody corresponding to each of the plurality of substances is arranged in the test line (detection part).

### (Test strip)

The test strip is preferably arranged on a solid phase support (backing sheet 1) such as a plastic-made pressure-sensitive adhesive sheet. The solid phase support is constituted of a substance that does not hinder the capillary flow of the sample and the conjugate. In addition, the test strip may be fixed on the solid phase support with an adhesive or the like.

The test strip includes a sample pad and an insoluble membrane carrier, and may further include other configurations depending on the measurement conditions and the sample. The other configurations are, for example, a conjugate pad, a third pad, an absorption pad, or the like. It is preferable that at least one of the sample pad, the conjugate pad, or the third pad of the test strip contain glass fibers. However, in a case where the test sample is filtered through a filter for specimen filtration containing glass fibers in the presence of the cationic substance before the test sample is brought into contact with the sample pad, none of the sample pad, the conjugate pad, and the third pad of the test strip may contain glass fibers.

Specifically, the test strip is the same as the reagent used in the test method according to the first embodiment described above.

### (Test kit)

A test kit for performing the test method according to the first embodiment includes the above-described reagents.

The test kit for performing the test method according to the first embodiment can also include a specimen filtration filter containing glass fibers, a cotton swab for sampling a specimen, a standard antigen substance, an antigen sample for accuracy control, or an instruction manual, in addition to the above-described reagents. The kit according to the embodiment of the present invention preferably includes a specimen filtration filter containing glass fibers. The specimen filtration filter is as described in the test method according to the embodiment of the present invention.

### <Second embodiment>

A reagent for performing the test method according to the second embodiment of the present invention is configured to be able to test one substance to be detected with one reagent. Specifically, the conjugate pad contains an antibody corresponding to one substance to be detected, and the test line (detection part) is arranged to correspond to one test substance.

A test kit for performing the test method according to the second embodiment of the present invention is different from the test kit for performing the test method according to the first embodiment of the present invention in that a reagent configured to be able to test one substance to be detected with one reagent instead of the reagent for performing the test method according to the first embodiment of the present invention is used, but is the same except for this point.

### [Examples]

Hereinafter, the present invention will be described in more detail with reference to examples, but is not limited to the examples described later in any way. Unless otherwise specified, "%" means "% by mass" .

### [Production Example 1] Production of immunochromatography test strip for SARS-CoV-2 measurement

### (1) Preparation of anti-SARS-CoV-2 monoclonal antibody

The anti-SARS-CoV-2 monoclonal antibody used in the following tests was obtained by immunizing a mouse with a recombinant SARS-CoV-2 nuclear protein as an antigen and using a method usually performed by those skilled in the art for manufacturing a monoclonal antibody.

### (2) Production of gold colloidal particle-labeled anti-SARS-CoV-2 monoclonal antibody solution

1 mL of a phosphate buffer containing 25 µg/mL of the anti-SARS-CoV-2 monoclonal antibody was added to 20 mL of a 1 OD/mL gold colloid solution, and the mixture was stirred at room temperature for 10 minutes. Subsequently, 2 mL of a 10% bovine serum albumin (BSA) solution was added to the gold colloid solution, and the mixture was stirred at room temperature for 5 minutes. The obtained solution was centrifuged at 10° C and 10,000 rpm for 45 minutes and the supernatant was removed. The residue was suspended in Conjugate Dilution Buffer (Scripps Laboratories) to obtain a gold colloid-labeled anti-SARS-CoV-2 monoclonal antibody solution.

### (3) Production of conjugate pad

The gold colloid-labeled anti-SARS-CoV-2 monoclonal antibody solution prepared in (2) was diluted with a 1.33% casein and 4% sucrose solution (pH 7.5) to 4 OD/mL to prepare a labeled reagent solution. The labeled reagent solution was applied to a glass fiber pad in a line shape, and the labeled reagent solution was dried to obtain a conjugate pad.

### (4) Production of antibody-immobilized membrane

Phosphate-buffered saline (PBS) containing 0.75 mg/mL of the anti-SARS-CoV-2 antibody and 2.5% sucrose was prepared to obtain a detection part coating liquid. PBS containing 1.0 mg/mL of a goat anti-mouse IgG monoclonal antibody and 2.5% sucrose was prepared to obtain a control part coating liquid. Using an immunochromatography dispenser (dispensing platform XYZ3050, BioDot company), the detection part coating liquid and the control part coating liquid were each applied onto a nitrocellulose membrane at 1.0 µL/cm, and then dried to obtain an antibody-immobilized membrane.

### (5) Production of test strip

The antibody-immobilized membrane, the conjugate pad, and the absorption pad were attached to a plastic-made pressure-sensitive adhesive sheet, and the resulting sheet was cut to a width of 5 mm to obtain an immunochromatography test strip for SARS-CoV-2 measurement.

### [Analysis Example 1] Evaluation of influence on sensitivity of antigen solution by filter filtration

The following antigens were each added to a specimen-diluting liquid included in an influenza virus kit (RapidTesta (registered trademark) FLU· NEXT, Sekisui Medical Company, Limited) to prepare a sample. · Influenza A virus (FLU A) antigen: derived from Kitakyusyu 159/93 strain
· Influenza B virus (FLU B) antigen: derived from Lee 40 strain
· RS virus antigen: RSV Antigen, Long strain inactivated antigen (Meridian Life Science, Inc.)
· Adenovirus antigen: Adenovirus Antigen (Fitzgerald Industries International)
· SARS-CoV-2 antigen: SARS-CoV-2 Nucleocapsid Recombinant Protein (Icosagen)

Part of the sample was filtered through a filter for specimen filtration included in RapidTesta FLU· NEXT to obtain a filtered sample. The sample and the filtered sample were measured under the conditions shown in Table 1, and the absorbance of the test line was measured with a densitometry analysis apparatus (RapidTesta (registered trademark) reader, Sekisui Medical Company, Limited).

**[Table 1]**

| Antigen | Measurement reagent | Amount of antigen solution | Measurement time |
|---|---|---|---|
| FLU A | RapidTesta FLU· NEXT test device | 120 *µ*L | 15 minutes |
| FLU B | RapidTesta FLU· NEXT test device | 120 *µ*L | 15 minutes |
| RS virus | RapidTesta RSV-Adeno· NEXT test device | 120 *µ*L | 10 minutes |
| Adenovirus | RapidTesta RSV-Adeno· NEXT test device | 120 *µ*L | 10 minutes |
| SARS-CoV-2 | Immunochromatography test strip for SARS-CoV-2 measurement | 120 *µ*L | 15 minutes |

A filtration recovery rate in a case where the sample was filtered through a filter for specimen filtration was calculated using the following expression.

Recovery rate (%) = absorbance in measurement of filtered sample/absorbance in measurement of sample (not filtered) × 100. Table 2 shows the filtration recovery rate of each antigen.

**[Table 2]**

| Antigen | Filtration recovery rate |
|---|---|
| Influenza A virus | 88% |
| Influenza B virus | 99% |
| RS virus | 91% |
| Adenovirus | 101% |
| SARS-CoV-2 | 47% |

In all of the FLU A antigen, the FLU B antigen, the RS virus antigen, and the adenovirus antigen, the filtration recovery rate was within 100 ± 15%, and no influence on the measurement value in a case where the sample was filtered through a filter for specimen filtration was observed. On the other hand, in the SARS-CoV-2 antigen, the filtration recovery rate was significantly low at 47% compared with other antigens, and the measurement value was decreased in a case where the sample was filtered using a filter for specimen filtration. In general, the specimen-diluting liquid for influenza virus measurement reagent and the filter for specimen filtration are also usable in the RS virus and adenovirus measurement reagent of the same series. However, it was suggested that in a case where the specimen-diluting liquid thereof and the filter for specimen filtration were applied to the SARS-CoV-2 measurement, there was a possibility that the highest sensitivity of the measurement reagent was not obtained.

### [Analysis Example 2] Evaluation of antigen adsorption to filter member

The SARS-CoV-2 antigen was added to a specimen-diluting liquid included in RapidTesta FLU· NEXT to prepare a sample. An opening side filter holding member (made of polyvinyl alcohol (PVA)), a filter member (made of glass fiber), and a distal end side filter holding member (made of PVA) were taken out from the filter for specimen filtration included in RapidTesta FLU· NEXT, and each was immersed in 500 µL of a sample for 10 minutes to obtain a filter member-immersed sample. 120 µL of the sample and the filter member-immersed sample were each added dropwise to an immunochromatography test strip for SARS-CoV-2 measurement. Fifteen minutes after dropping the antigen solution, the absorbance of the test line was measured with a RapidTesta reader (Sekisui Medical Company, Limited).

The recovery rate of the antigen in a case where the filter member for specimen filtration was immersed in a sample was calculated using the following expression. Recovery rate (%) = absorbance in measurement of filter member-immersed sample/absorbance in measurement of sample (filter member was not immersed) × 100

Table 3 shows the recovery rate of the antigen in a case where each member was immersed in a sample.

**[Table 3]**

| Filter member for specimen filtration | Material of filter member | Recovery rate |
|---|---|---|
| Opening side filter holding member | PVA | 74% |
| Filter material | Glass fiber | 10% |
| Distal end side filter holding member | PVA | 69% |
| All filter member | PVA and Glass fiber | 9% |

The recovery rate in a case where the filter member made of glass fiber was immersed in the sample was approximately 10%, whereas the recovery rate of the antigen in a case where the filter holding member made of PVA was immersed in the sample was approximately 70%. Therefore, it was suggested that in the SARS-CoV-2 measurement, in a case where a diluting liquid for the influenza virus measurement reagent and a filter made of glass fiber were used, there was a possibility that the highest sensitivity of the measurement reagent was not obtained. In addition, the reason the filtration recovery rate of the SARS-CoV-2 antigen was remarkably low in Analysis Example 1 was considered to be that the SARS-CoV-2 antigen was adsorbed to the glass fibers.

### [Analysis Example 3] Evaluation of influence on virus antigen measurement sensitivity by sample pad member

A sample was prepared in the same manner as in Analysis Example 1. A sample pad member having an area of 140 mm² per 120 µL of the sample was immersed in the sample for 10 minutes to obtain a pad member-immersed sample. The sample and the pad member-immersed sample were measured under the conditions shown in Table 1, and the absorbance of the test line was measured with a RapidTesta reader (Sekisui Medical Company, Limited). The recovery rate of the antigen in a case where the pad member was immersed in a sample was calculated using the following expression. Recovery rate (%) = absorbance in measurement of pad member-immersed sample/absorbance in measurement of sample × 100

Table 4 shows the recovery rate of the antigen in a case where each member was immersed in a sample.

**[Table 4]**

| Antigen | Recovery rate of antigen | | | |
|---|---|---|---|---|
| | Glass fiber pad 1 | Glass fiber pad 2 | Polyester fiber pad 1 | Polyester fiber pad 2 |
| FLU A | 89% | 92% | 114% | 100% |
| FLU B | 95% | 106% | 106% | 100% |
| RS virus | 94% | 110% | 101% | 107% |
| Adenovirus | 107% | 111% | 93% | 102% |
| SARS-CoV-2 | 51% | 81% | 102% | 104% |

In the FLU A, FLU B, RS virus, and adenovirus antigens, also in a case where any of the pad members were immersed in the sample, the recovery rate of the antigen was within 100% ± 15%, which was favorable. On the other hand, in the SARS-CoV-2 antigen, the recovery rate of the antigen was significantly decreased in a case where the glass fiber pad was immersed in the sample.

From the results of Analysis Example 2 and Analysis Example 3, it was considered that, in the SARS-CoV-2, the amount of the antigen to be subjected to the subsequent immunochromatography was reduced and the recovery rate was decreased by the contact between the antigen and the glass-made member.

Here, Table 5 shows the isoelectric point of the protein to be detected by each measurement reagent included in each antigen used in Analysis Example 3 and an antigen similar thereto. The isoelectric point of each protein was calculated by inputting UniProtKB into Expaxy Compute pI/Mw (https://www.expasy.org/resources/computepi-mw).

**[Table 5]**

| Antigen | Protein detected by reagent | UniProtKB | Isoelectric point |
|---|---|---|---|
| Influenza A virus | Nucleocapsid protein | 091743 (NCAP_I93A0) | 9.35 |
| Influenza B virus | Nucleocapsid protein | P04665 (NCAP_INBLE) | 9.4 |
| RS virus | Fusion glycoprotein F0 | P03420 (FUS_HRSVA) | 9.1 |
| Adenovirus | Hexon protein | P03277 (CAPSH_ADE02) | 5.06 |
| SARS-CoV-2 | Nucleocapsid protein | P0DTC9 (NCAP_SARS2) | 10.07 |
| MERS-CoV | Nucleocapsid protein | K9N4V7 (NCAP_MERS1) | 10.05 |
| SARS-CoV | Nucleocapsid protein | P59595 (NCAP_SARS) | 10.11 |

It can be seen that only the isoelectric point of the nucleocapsid protein of SARS-CoV-2 in Table 5 is high, 10.0 or more. While the glass fibers have a negative charge in a case of being in contact with a neutral specimen-diluting liquid, the nucleocapsid protein of SARS-CoV-2 having an isoelectric point of 10.07 has a positive charge. Therefore, it is considered that the nucleocapsid protein of SARS-CoV-2 is easily adsorbed to the glass fibers compared with other antigen proteins having an isoelectric point of less than 10. On the other hand, it is considered that since PVA or polyester does not have a charge in a case of being in contact with the neutral specimen-diluting liquid, the adsorption of the antigen protein to the pad is not allowed regardless of the isoelectric point of the antigen protein.

### [Example 1]

### · Influence of cationic substance on SARS-CoV-2 antigen measurement sensitivity

As a sample, a mixture obtained by adding each of the cationic substances shown in Table 6 and SARS-CoV-2 Nucleocapsid Recombinant Protein (Icosagen) at a concentration of 450 TCID50/mL equivalent to a specimen-diluting liquid solution included in RapidTesta FLU· NEXT was used. 120 µL of each sample was added dropwise to the immunochromatography test strip for SARS-CoV-2 measurement produced by the method described in Production Example 1. Ten minutes after adding the antigen solution, the absorbance of the test line was measured with a RapidTesta reader (Sekisui Medical Company, Limited). The configuration of the immunochromatography test strip used was the same as that in FIG. 2.

Table 6 shows the results.

**[Table 6]**

| Type of cationic substance | Cationic substance (final concentration) | | Absorbance [mAbs] |
|---|---|---|---|
| - | None | | 49.8 |
| Metal salt | 150 mM | NaCl | 71.9 |
| | 250 mM | NaCl | 81.4 |
| | 10 mM | Trisodium citrate | 72.2 |
| | 100 mM | Trisodium citrate | 102.4 |
| | 5 mM | MgSO₄ | 94.7 |
| | 10 mM | MgSO₄ | 111.6 |
| Cationic polymer | 0.0005% | Hexadimethrine bromide | 65.6 |
| | 0.001% | Hexadimethrine bromide | 103.7 |
| | 0.01% | Hexadimethrine bromide | 104.3 |
| | 0.03% | Hexadimethrine bromide | 84 |
| | 0.01% | Lipidure (registered trademark) BL-502 | 78.7 |
| | 0.05% | Lipidure BL-502 | 90.2 |
| | 0.20% | Lipidure BL-502 | 106 |
| Cationic surfactant | 0.50% | QUARTAMINE (registered trademark) 24P | 116.7 |
| | 1.00% | QUARTAMINE 24P | 169.4 |
| | 0.05% | CTAB | 143.5 |
| | 0.10% | CTAB | 97.4 |
| Cationic peptide | 0.50% | Promois WK-HCAQ-NA | 72.6 |
| | 1.00% | Promois WK-HCAQ-NA | 69.5 |
| Cationic polymer + metal salt | 0.05% 250 mM | Lipidure BL-502 NaCl | 244.3 |
| "Promois" is registered trademark. | | | |

In all conditions in which the cationic substance was added, the absorbance in the measurement of the SARS-CoV-2 antigen was increased compared with the condition in which the additive was not added. It is considered that since the immunochromatography test strip for SARS-CoV-2 measurement includes a glass fiber pad, adsorption of the SARS-CoV-2 antigen to the glass fibers is suppressed by the cationic substance.

### · Influence of cationic substance on recovery rate of SARS-CoV-2 antigen

As a sample, a mixture obtained by adding each of the cationic substances shown in Table 7 and SARS-CoV-2 Nucleocapsid Recombinant Protein (Icosagen) at a concentration of 450 TCID50/mL equivalent to a specimen-diluting liquid solution included in RapidTesta FLU· NEXT was used.

The sample was filtered through a filter for specimen filtration included in RapidTesta FLU· NEXT to obtain a filtered sample.

120 µL of the sample and the filtered sample were each added dropwise to the immunochromatography test strip for SARS-CoV-2 measurement produced by the method described in Production Example 1. Ten minutes after adding the antigen solution, the absorbance of the test line was calculated with a RapidTesta reader (Sekisui Medical Company, Limited). Each sample was measured three times. The configuration of the immunochromatography test strip used was the same as that in FIG. 2.

A filtration recovery rate in a case where the sample was filtered through a firlter for specimen filtration was calculated using the following expression. Recovery rate (%) = absorbance in measurement of filtered sample/absorbance in measurement of sample (not filtered) × 100.

**[Table 7]**

| Condition | No additives | | 0.05% Lipidure502 | | 250 mM NaCl | | 0.05% Lipidure-BL502 + 250 mM NaCl | |
|---|---|---|---|---|---|---|---|---|
| Filter filtration | Absence | Presence | Absence | Presence | Absence | Presence | Absence | Presence |
| First time | 38.9 | 9.8 | 85.0 | 43.8 | 85.8 | 75.9 | 116.5 | 120.0 |
| Second time | 55.0 | 12.4 | 93.3 | 35.8 | 102.2 | 67.8 | 120.2 | 121.7 |
| Third time | 29.7 | 15.3 | 103.0 | 47.7 | 93.4 | 80.9 | 128.7 | 121.8 |
| Average value | 41.20 | 12.50 | 93.76 | 42.43 | 93.82 | 74.84 | 121.82 | 121.17 |
| Recovery rate | - | 30.3% | - | 45.3% | - | 79.8% | - | 99.5% |

The recovery rate of the antigen was improved by performing the filtration using a glass fiber filter in the presence of a cationic substance.

### [Production Example 2] Production of immunochromatography test strip for measuring SARS-CoV-2/FLU

### (1) Preparation of anti-FLU monoclonal antibody

The anti-FLU A monoclonal antibody and the anti-FLU B monoclonal antibody used in the following tests were obtained by immunizing a mouse with a FLU A or FLU B antigen and by a method usually performed by those skilled in the art for manufacturing a monoclonal antibody.

### (2) Production of gold colloid-labeled anti-FLU antibody solution

A gold colloid-labeled anti-FLU A antibody solution and a gold colloid-labeled anti-FLU B antibody solution were obtained by the same operation as in Production Example 1.

### (3) Production of conjugate pad

A 1.33% casein and 4% sucrose solution (pH 7.5) was prepared in which the gold colloid-labeled anti-SARS-CoV-2 monoclonal antibody solution adjusted as in Production Example 1, the gold colloid-labeled anti-FLU A antibody solution prepared in 2, and the gold colloid-labeled anti-FLU B antibody solution was added at a concentration of 4 OD/mL, and used as a labeled reagent solution. The labeled reagent solution was applied to the glass fiber pad in a line shape, and the labeled reagent solution was dried to obtain a conjugate pad.

### (4) Production of antibody-immobilized membrane

A PBS containing 0.75 mg/mL of the anti-SARS-CoV-2 antibody and 2.5% sucrose was prepared and used as a SARS-CoV-2 test line coating liquid. Similarly, the FLU A detection part coating liquid and the FLU B detection part coating liquid were prepared. PBS containing 1.0 mg/mL of a goat anti-mouse IgG monoclonal antibody and 2.5% sucrose was prepared and used as a control part coating liquid. Using an immunochromatography dispenser "XYZ3050" (BioDot company), the detection part coating liquid and the control part coating liquid were each applied onto a nitrocellulose membrane at 1.0 µL/cm, and the membrane was dried to obtain an antibody-immobilized membrane. Each line was arranged in the order of the FLU A detection part, the FLU B detection part, the SARS-CoV-2 detection part, and the control part from the upstream in the specimen development direction.

### (5) Production of test strip

The antibody-immobilized membrane, the conjugate pad, and the absorption pad were attached to a plastic-made pressure-sensitive adhesive sheet, and the resulting sheet was cut to a width of 5 mm to obtain an immunochromatography test strip for measuring SARS-CoV-2/FLU.

### [Example 2]

### · Influence of cationic substance on each antigen measurement sensitivity <1>

A SARS-CoV-2 positive specimen, a FLU A positive specimen, and a FLU B positive specimen were each suspended in a specimen-diluting liquid included in RapidTesta FLU· NEXT and a solution prepared by adding the cationic substance shown in Tables 7 to 9 to a specimen-diluting liquid included in RapidTesta FLU· NEXT, and used as a sample. The distal end of the immunochromatography test strip for measuring SARS-CoV-2/FLU on the conjugate pad side was immersed in the sample, and the absorbance of the test line was measured 10 minutes later using a RapidTesta reader (Sekisui Medical Co., Ltd.).

The results are shown in Tables 8, 9, and 10.

**[Table 8]**

| SARS-CoV-2 detection part | | | | |
|---|---|---|---|---|
| Specimen | Cationic substance | | | |
| | Absence | 0.05% Lipidure BL-502 | 250 mM NaCl | 0.05% Lipidure BL-502 + 250 mM NaCl |
| SARS-CoV-2 positive specimen 1 | + | + | + | 2+ |
| SARS-CoV-2 positive specimen 2 | + | 2+ | + | 2+ |
| SARS-CoV-2 positive specimen 3 | + | 2+ | 2+ | 3+ |
| SARS-CoV-2 positive specimen 4 | + | + | + | 2+ |
| SARS-CoV-2 positive specimen 5 | + | 2+ | 2+ | 2+ |
| SARS-CoV-2 positive specimen 6 | 2+ | 2+ | 2+ | 2+ |
| SARS-CoV-2 positive specimen 7 | 2+ | 3+ | 2+ | 3+ |
| SARS-CoV-2 positive specimen 8 | 2+ | 3+ | 2+ | 3+ |
| SARS-CoV-2 positive specimen 9 | 3+ | 5+ | 5+ | 5+ |
| SARS-CoV-2 positive specimen 10 | 4+ | 4+ | 4+ | 4+ |
| SARS-CoV-2 positive specimen 11 | 4+ | 5+ | 5+ | 5+ |
| SARS-CoV-2 positive specimen 12 | 4+ | 5+ | 5+ | 5+ |
| SARS-CoV-2 positive specimen 13 | 5+ | 5+ | 5+ | 5+ |

The determination was made as follows according to the absorbance of the test line.
-: less than 5 mAbs (negative)
+: less than 20 mAbs (positive)
2+: 20 mAbs or more and less than 100 mAbs (positive)
3+: 100 mAbs or more and less than 200 mAbs (positive)
4+: 200 mAbs or more and less than 400 mAbs (positive)
5+: 400 mAbs or more (positive)

**[Table 9]**

| FLU A detection part | | | | |
|---|---|---|---|---|
| Specimen | Cationic substance | | | |
| | Absence | 0.05% Lipidure BL-502 | 250 Mm NaCl | 0.05% Lipidure BL-502 + 250 mM NaCl |
| FLU A positive specimen 1 | 4+ | 4+ | 4+ | 4+ |
| FLU A positive specimen 2 | 2+ | 2+ | 2+ | 2+ |
| FLU A positive specimen 3 | 5+ | 5+ | 5+ | 5+ |
| FLU A positive specimen 4 | 2+ | 2+ | 2+ | 2+ |
| FLU A positive specimen 5 | 3+ | 3+ | 3+ | 3+ |

The determination was made as follows according to the absorbance of the test line.
-: less than 5 mAbs (negative)
+: less than 20 mAbs (positive)
2+: 20 mAbs or more and less than 100 mAbs (positive)
3+: 100 mAbs or more and less than 200 mAbs (positive)
4+: 200 mAbs or more and less than 400 mAbs (positive)
5+: 400 mAbs or more (positive)

**[Table 10]**

| FLU B detection part | | | | |
|---|---|---|---|---|
| Specimen | Cationic substance | | | |
| | Absence | 0.05% Lipidure BL-502 | 250 mM NaCl | 0.05% Lipidure BL-502 + 250 mM NaCl |
| FLU B positive specimen 1 | 2+ | 3+ | 2+ | 2+ |
| FLU B positive specimen 2 | 3+ | 3+ | 3+ | 3+ |
| FLU B positive specimen 3 | 2+ | 2+ | 2+ | 2+ |
| FLU B positive specimen 4 | 5+ | 5+ | 5+ | 5+ |
| FLU B positive specimen 5 | 3+ | 3+ | 3+ | 3+ |

The determination was made as follows according to the absorbance of the test line.
-: less than 5 mAbs (negative)
+: less than 20 mAbs (positive)
2+: 20 mAbs or more and less than 100 mAbs (positive)
3+: 100 mAbs or more and less than 200 mAbs (positive)
4+: 200 mAbs or more and less than 400 mAbs (positive)
5+: 400 mAbs or more (positive)

In the measurement of the SARS-CoV-2 positive specimen, the measurement values of all the specimens were increased by the cationic substance. Even in a case of the same determination, the absorbance of the condition in which the cationic substance was added was higher than the absorbance of the non-addition condition in all the specimens. In the measurement of the FLU A positive specimen, the tendency to increase the measured value due to the addition of the cationic substance was not recognized. In the FLU B positive specimen, the tendency of the measurement value variation due to the cation additive varied depending on the specimen, but a positive determination was made in all the conditions and specimens, and no adverse effect on the measurement accuracy due to the cationic substance was recognized. As shown in Analysis Example 3, it is considered that the difference in the antigen adsorptivity to the glass fibers between the SARS-CoV-2 antigen and the FLU antigen causes the difference in the effect of the cationic substance. No specimen in which a plurality of test lines were positive was recognized.

### [Example 3]

### · Influence of cationic substance on each antigen measurement sensitivity <2>

The RS virus antigen, the adenovirus antigen, and the SARS-CoV-2 antigen described in Analysis Example 1 were each added to a specimen-diluting liquid included in RapidTesta FLU· NEXT and a solution prepared by adding the cationic substance shown in Tables 11 to 12 to a specimen-diluting liquid included in RapidTesta FLU· NEXT, and used as a sample. The prepared sample was measured using the immunochromatography test strip for SARS-CoV-2 measurement prepared in the same manner as in Production Example 1 and RapidTesta RSV-Adeno· NEXT (manufactured by Sekisui Medical Company, Limited). Absorbance of the test line of each reagent was measured using a RapidTesta reader (Sekisui Medical Company, Limited). The test was performed three times for each antigen, and the measurement results are shown in absorbance (unit: mAbs). In a case where the absorbance was 5.0 mAbs or less, which is the measurement lower limit of the RapidTesta reader, it was determined to be negative, and "<5.0" is shown in the table.

The results are shown in Tables 11 and 12.

**[Table 11]**

| Test strip | Immunochromatography test strip for SARS-CoV-2 measurement | | | |
|---|---|---|---|---|
| Cationic additive | Absence | 0.05% Lipidure BL-502 | 250 mM NaCl | 0.05% Lipidure BL-502 + 250 mM NaCl |
| Specimen-diluting liquid | <5.0 | <5.0 | <5.0 | <5.0 |
| | <5.0 | <5.0 | <5.0 | <5.0 |
| | <5.0 | <5.0 | <5.0 | <5.0 |
| SARS-CoV-2 antigen 450 TCID50/mL equivalent | 45.3 | 75.2 | 78.8 | 92.4 |
| | 36.7 | 70.3 | 84.3 | 87.7 |
| | 34.7 | 72.6 | 81.5 | 88.3 |

**[Table 12]**

| Test strip | RapidTesta RSV-Adeno· NEXT | | | | | | | |
|---|---|---|---|---|---|---|---|---|
| Cationic additive | Absence | | 0.05% Lipidure BL-502 | | 250 mM NaCl | | 0.05% Lipidure BL-502 + 250 mM NaCl | |
| Test line | RS | Adeno | RS | Adeno | RS | Adeno | RS | Adeno |
| Specimen-diluting liquid | <5.0 | <5.0 | <5.0 | <5.0 | <5.0 | <5.0 | <5.0 | <5.0 |
| | <5.0 | <5.0 | <5.0 | <5.0 | <5.0 | <5.0 | <5.0 | <5.0 |
| | <5.0 | <5.0 | <5.0 | <5.0 | <5.0 | <5.0 | <5.0 | <5.0 |
| RS virus antigen 2.0 × 10⁴ TCID50/mL | 37 | <5.0 | 38.3 | <5.0 | 35.3 | <5.0 | 37.2 | <5.0 |
| | 34.8 | <5.0 | 33.5 | <5.0 | 37.7 | <5.0 | 35.5 | <5.0 |
| | 36.3 | <5.0 | 36.8 | <5.0 | 36.4 | <5.0 | 35.8 | <5.0 |
| Adenovirus antigen 2.5 × 10⁴ TCID50/mL | <5.0 | 47.1 | <5.0 | 48.9 | <5.0 | 43 | <5.0 | 53.1 |
| | <5.0 | 47 | <5.0 | 47.5 | <5.0 | 43.3 | <5.0 | 47.1 |
| | <5.0 | 48.5 | <5.0 | 51.9 | <5.0 | 43.8 | <5.0 | 51.8 |

In the measurement of the SARS-CoV-2 antigen, the absorbance was increased by the cationic additive. On the other hand, no similar absorbance variation was observed in the RS virus and the adenovirus. For any of the antigens, the negative sample and the positive sample were correctly determined without depending on the addition of the cation additive, and the cation additive which increased the measurement sensitivity of the antigen having an isoelectric point of 10.0 or more did not adversely affect the measurement result in a case where the cation additive coexisted in the measurement of the antigen having an isoelectric point of less than 10.

### [Industrial Applicability]

In the test method and test kit according to the embodiments of the present invention, since it is not necessary to collect a specimen from the nasal cavity or the pharynx for each pathogen of a test target, the risk of droplet exposure to a medical worker by a cough or a sneeze of a patient can be reduced. Therefore, it is possible to perform more safely a test for specifically identifying the causative virus of the infection.

### [Reference Signs List]

1: Backing sheet
2: Conjugate pad
3: Absorption pad
4: Insoluble membrane carrier
5: Labeled reagent holding part
6: Test line (Detection part)
7: Control line
8: Sample pad
9: Third pad

## Claims

1. A qualitative or quantitative test method to detect a substance to be detected contained in a specimen, the test method comprising:
a preparation step of preparing a test sample from the specimen; and
a test step of testing at least part of the test sample with a reagent to detect each of a first substance to be detected and a second substance to be detected,
wherein the first substance to be detected has an isoelectric point of 10.0 or more, and
in any one or both of the preparation step and the test step, the first substance to be detected is brought into contact with any one or both of a glass-made member and a nitrocellulose-made member in a presence of a cationic substance.

2. The test method according to Claim 1,
wherein the reagent includes a sample supply part and a labeled reagent holding part, and any one or both of the sample supply part and the labeled reagent holding part include the glass-made member.

3. A qualitative or quantitative test method to detect a substance to be detected contained in a specimen, the test method comprising:
a preparation step of preparing a test sample from the specimen;
a first test step of testing at least part of the test sample with a first reagent to detect each of a first substance to be detected and a second substance to be detected; and
a second test step of testing part of the test sample with a second reagent to detect the second substance to be detected,
wherein the first substance to be detected has an isoelectric point of 10.0 or more, and
in any one or both of the preparation step and the first test step, the first substance to be detected is brought into contact with any one or both of a glass-made member and a nitrocellulose-made member in a presence of a cationic substance.

4. The test method according to Claim 3,
wherein the first reagent includes a sample supply part and a labeled reagent holding part, and any one or both of the sample supply part and the labeled reagent holding part include the glass-made member.

5. The test method according to any one of Claims 1 to 4,
wherein the preparation step includes a step of suspending the specimen in a specimen-diluting liquid.

6. The test method according to Claim 5,
wherein the cationic substance is contained in the specimen-diluting liquid.

7. The test method according to any one of Claims 1 to 4,
wherein the preparation step includes a step of filtering the test sample with a filter.

8. The test method according to any one of Claims 1 to 4,
wherein the glass-made member is a filter for specimen filtration including glass fibers.

9. The test method according to any one of Claims 1 to 4,
wherein the cationic substance is at least one selected from the group consisting of a metal salt, a cationic polymer, a cationic peptide, and a cationic surfactant.

10. The test method according to any one of Claims 1 to 4,
wherein the substance having an isoelectric point of 10.0 or more is derived from a respiratory infection virus.

11. The test method according to Claim 10,
wherein the respiratory infection virus is a coronavirus.

12. The test method according to Claim 11,
wherein the coronavirus is SARS-CoV-2.

13. A reagent comprising:
a first labeled reagent in which a first binding substance capable of binding to a first substance to be detected is labeled with a labeling substance;
a second labeled reagent in which a second binding substance capable of binding to a second substance to be detected is labeled with a labeling substance;
a first detection part in which a first solid phase reagent capable of binding to the first substance to be detected is immobilized and a second detection part in which a second solid phase reagent capable of binding to the second substance to be detected is immobilized; and
any one or both of a glass-made member and a nitrocellulose-made member arranged upstream of the first detection part in a development direction of a development liquid containing a specimen,
wherein the first substance to be detected has an isoelectric point of 10.0 or more, and
the development liquid brought into contact with any one or both of the glass-made member and the nitrocellulose-made member contains a cationic substance.

14. The reagent according to Claim 13,
wherein the reagent includes a sample supply part and a labeled reagent holding part, and any one or both of the sample supply part and the labeled reagent holding part include the glass-made member.

15. The reagent according to Claim 13 or 14,
wherein the glass-made member is a filter for specimen filtration including glass fibers.

16. The reagent according to Claim 13 or 14,
wherein the substance having an isoelectric point of 10.0 or more is derived from a respiratory infection virus.

17. The reagent according to Claim 16,
wherein the respiratory infection virus is a coronavirus.

18. The reagent according to Claim 17,
wherein the coronavirus is SARS-CoV-2.

19. The reagent according to Claim 13 or 14,
wherein the cationic substance is at least one selected from the group consisting of a metal salt, a cationic polymer, a cationic peptide, and a cationic surfactant.

20. A qualitative or quantitative test method of a substance to be detected contained in a specimen using the reagent according to Claim 13 or 14.

21. A test kit comprising:
a reagent,
wherein the reagent includes a first labeled reagent in which a first binding substance capable of binding to a first substance to be detected is labeled with a labeling substance, a second labeled reagent in which a second binding substance capable of binding to a second substance to be detected is labeled with a labeling substance, a first detection part in which a first solid phase reagent capable of binding to the first substance to be detected is immobilized, and a second detection part in which a second solid phase reagent capable of binding to the second substance to be detected is immobilized,
the test kit includes any one or both of a glass-made member and a nitrocellulose-made member arranged upstream of the first detection part in a development direction of a development liquid containing a specimen,
the first substance to be detected has an isoelectric point of 10.0 or more, and
the development liquid brought into contact with any one or both of the glass-made member and the nitrocellulose-made member contains a cationic substance.

22. A test kit comprising:
a specimen filtration filter; and
a reagent,
wherein the reagent includes a first labeled reagent in which a first binding substance capable of binding to a first substance to be detected is labeled with a labeling substance, a second labeled reagent in which a second binding substance capable of binding to a second substance to be detected is labeled with a labeling substance, a first detection part in which a first solid phase reagent capable of binding to the first substance to be detected is immobilized, and a second detection part in which a second solid phase reagent capable of binding to the second substance to be detected is immobilized,
the test kit includes any one or both of a glass-made member and a nitrocellulose-made member arranged upstream of the first detection part in a development direction of a development liquid containing any one or both of a specimen of the specimen filtration filter and a specimen of the reagent,
the first substance to be detected has an isoelectric point of 10.0 or more, and
the development liquid brought into contact with any one or both of the glass-made member and the nitrocellulose-made member contains a cationic substance.

23. The test kit according to Claim 21 or 22,
wherein the reagent includes a sample supply part and a labeled reagent holding part, and any one or both of the sample supply part and the labeled reagent holding part include the glass-made member.

24. The test kit according to Claim 21 or 22,
wherein the glass-made member is a filter for specimen filtration including glass fibers.

25. The test kit according to Claim 21 or 22,
wherein the substance having an isoelectric point of 10.0 or more is derived from a respiratory infection virus.

26. The test kit according to Claim 25,
wherein the respiratory infection virus is a coronavirus.

27. The test kit according to Claim 26,
wherein the coronavirus is SARS-CoV-2.

28. The test kit according to Claim 21 or 22, further comprising:
a specimen-diluting liquid.

29. The test kit according to Claim 28,
wherein the cationic substance is contained in the specimen-diluting liquid.

30. The test kit according to Claim 21 or 22,
wherein the cationic substance is at least one selected from the group consisting of a metal salt, a cationic polymer, and a cationic surfactant.

31. A qualitative or quantitative test method of a substance to be detected contained in a specimen, using the test kit according to Claim 21 or 22.
